(19) Europäisches Patentamt
European Patent Office
Office européen des brevets

(11) Publication number: 0 177 071 B1

# (12) EUROPEAN PATENT SPECIFICATION

(45) Date of publication of patent specification: 02.08.89

(51) Int. Cl.[4]: C 11 D 1/37, A 61 K 7/08, A 61 K 7/50

(21) Application number: 85201309.3

(22) Date of filing: 14.08.85

## (54) Mixture of alkylether sulphate and alkylpolyglycol ether carboxylic acid or salt thereof.

(30) Priority: 23.08.84 NL 8402578

(43) Date of publication of application:
09.04.86 Bulletin 86/15

(45) Publication of the grant of the patent:
02.08.89 Bulletin 89/31

(84) Designated Contracting States:
AT BE CH DE FR GB IT LI LU NL SE

(56) References cited:
FR-A-2 338 326
GB-A- 874 186

SEIFEN-OLE-FETTE-WACHSE, vol. 109, no. 13, August 1983, pages 353-355, Augsburg, DE; N.A.I. VAN PAASSEN: "Alkylethercarboxylate: Hautfreundliche Rohstoffe für kosmetische Anwendungen"

CHEMICAL ABSTRACTS, vol. 77, no. 14, 2nd October 1977, page 121, abstract no. 90418j, Columbus, Ohio, US

CHEMICAL ABSTRACTS, vol. 81, 1974, page 133, abstract no. 107828a, Columbus, Ohio, US

(73) Proprietor: STAMICARBON B.V.
Mijnweg 1
NL-6167 AC Geleen (NL)

(72) Inventor: Smid, Jacob Cornelis
J.G. Heuthorststraat 61
Doetinchem (NL)
Inventor: van Paassen, Nicolaas Adrianus Ignatius
Elzenhof 11
Bodegraven (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 177 071 B1

**Description**

This invention relates to a mixture of alkylether sulphate and alkylpolyglycol ether carboxylic acid or salt thereof, particularly suitable for frequent or prolonged contact with human hair and skin. Consequently, such mixtures are particularly suitable for use in shampoos, shower and bath compositions, liquid soap particularly for hand-washing, and compositions for non-automatic dishwashing.

Mixtures of alkylether sulphates and alkylpolyglycol ether carboxylic acids or salts thereof are known per se already from British patent 874,186. It appears from this patent that this kind of mixtures shows a number of synergistic effects which make them particularly suitable for use in shampoos. In the meantime this kind of mixtures has been used already on a large scale in shampoos, showering compositions and the like. The number of oxyethylene units in the ether carboxylic component therein is generally small, particularly less than 10.

A similar kind of mixture in combination with a cosmetically acceptable oil is disclosed in French patent 2,338,326. According to page 4 thereof, the ether carboxylate should constitute the major component of the mixture thereof with the ether sulphate.

In an article by N. A. I. van Paassen in Seifen, Ölen, Fette, Wachse, Heft 13/1983, pages 353—55, it has been shown that the skin toxicity of alkylpolyglycol ether carboxylates having an average of 10 or more oxyethylene units is lower than that of lower compounds from this series and that mixtures of such highly ethoxylated carboxylates with sodium laurylether sulphate having an average of 2 oxyethylene groups show a high decrease of skin toxicity. In this work mixtures have been tested having a minimum of 25% ether carboxylate and at the end of the article it is mentioned as a practical possibility to replace about 25% of the sodium laurylether sulphate with an ether carboxylic acid.

It has now been found that the synergistic toxicity effect is already so clearly appreciable at much lower percentages that much lower contents of polyglycolether carboxylic acid can suffice. Otherwise stated, the toxicity curve is particularly steep, exactly in the area below 25% of polyglycolether carboxylic acid, which means that exactly in that area the skin toxicity decreases to a relatively much stronger degree than in the area tested according to the article in Seifen, Öle, Fette, Wachse. In actual practice this means that with much lower percentages of alkylpolyglycolether carboxylic acid mixtures are obtained which possess a considerably lower skin toxicity than the mixtures which are usual in practice and which contain alkylglycol ether carboxylic acid having a low number of oxyethylene units.

Accordingly, the invention provides a mixture of alkylether sulphate and alkylpolyglycolether carboxylic acid or a salt thereof, particularly suitable for frequent or prolonged contact with human hair and skin, wherein the alkylpolyglycol ether carboxylic acid contains at least 7 oxyethylene units, said mixture being characterized by the fact that it contains 80—95% by weight of alkylether sulphate and 20—5% by weight of alkylpolyglycol ether carboxylic acid or salt thereof of the formula

$$RO—(C_2H_4O)_x—CH_2COOM$$

wherein R is an alkyl residue having 10—20 carbon atoms, x is a number having an average value of 7.25 and M represents hydrogen, sodium, potassium, magnesium or an amine salt.

As alkylether sulphate one can use all the compounds usual for this purpose. A commercial product which is very frequently used in shampoos and the like is a lauryl ether sulphate, wherein the lauryl residue may be replaced for up to about 30% with myristyl, the number of oxyethylene units in this product generally being an average of about 2 to 3. In the examples herein below the invention will be elucidated with the aid of the use of this product, but only because this is the most usual commercial product. It will be evident that the invention is not restricted thereto. Furthermore, this lauryl ether sulphate conventionally is used as the sodium salt, but it is a matter of course that other salts can also be used, for instance potassium, magnesium and amine salts, although these are more expensive.

The alkylpolyglycol ether carboxylic acid is also preferably used in the form of the sodium salt, because no particular advantages are obtained by using other and more expensive salts. These polyether carboxylic acids can be represented by the general formula

$$RO—(C_2H_4O)_x—CH_2COOM,$$

wherein R is a usual high alkyl residue having 10—20 carbon atoms, preferably 12—18 carbon atoms, and x in this case is a number having an average value of 7—25, preferably 9—18 and M represents hydrogen, sodium, potassium, magnesium or an amine salt.

These ether carboxylic acids are known already as a class for a number of decades and their preparation has been disclosed in several patents. In connection with the principally cosmetic uses which are aimed at by the present invention, it is not the entire class of these compounds which can be used. Thus, the compounds, wherein R is an alkaryl group, generally are not suitable for cosmetic purposes, because their toxicity for the skin flora is too high. Furthermore, the low toxicity is only obtained with the compounds, wherein x has an average value of at least 7.

The introduction of the oxyethylene units can be carried out in the usual way, either with a basic catalyst (NaOH), or with a Lewis acid ($SbCl_5$). In the last mentioned case a mixture is obtained having a

narrower distribution of the number of oxyethylene units than in the first case. However, with products having these relatively high values of x, the narrow distribution has less advantages than with a product having low values of x. In the case of the preparation of an ether carboxylic acid having only one or two oxyethylene units, the oxyethylation product of the starting alcohol in the case of basic catalysis still contains a considerable amount of non-ethoxylated product, which in the subsequent reaction with haloacetic acid accordingly yields a compound $ROCH_2COOH$, which does not contribute much to the surfactant properties. In that case a superior product can be obtained with a catalyst like $SbCl_5^-$. However, in the preparation of products having a high x-value, no unethoxylated compound will occur anymore in the product, even when using basic catalysis.

Alkylpolyglycol ether carboxylic acids having high x-values possess a somewhat lower foaming capacity. This also appears from the above cited article in Seifen, Öle, Fette, Wachse. Exactly for shampoos, showering compositions and the like the consumer wishes a foaming capacity which is as high as possible and for that reason it is a particular advantage that for attaining an acceptable low skin toxicity a considerably lower amount of alkylpolyglycol ether carboxylic acid will suffice than was considered necessary according to the above mentioned article.

The following, non-restricting experiments elucidate the invention.

The experiments were carried out according to the so-called "patch-test method". The objective thereof was to measure the primary skin irritation by the substances concerned, compared to a control. The test was carried out with a group of 25 men and women in the ages between 16 and 65.

The test and control samples were applied to the skin of the upper arm with the aid of an occlusive "finn chamber". This is an occlusive aluminium disc which provides a test area of 50 $mm^2$ with a diameter of 8 mm and a volume of 25 µl. They are arranged on a non-occlusive backing tape at a standard space of 1 cm. These tapes are left in contact with the skin for 24 hours. Thereafter they are removed and the skin reaction is assessed one hour later. Immediately after this assessment a second identical tape with the same substance is applied to the same place on the skin and again left in contact with the skin for 24 hours. Again the reaction is assessed one hour after removal. The materials to be tested were randomly distributed over the several volunteers. All assessments were carried out under standard lighting conditions by a skilled assessor who was not aware which samples had been applied to which person.

The degree of irritation is expressed according to a score varying from 0 to 5. Therein these numbers have the following meanings:

0=no visable reaction;
1=reaction just present;
2=slight reaction;
3=moderate reaction;
4=severe reaction;
5=very severe reaction.

Furthermore, rating of significance have also been assigned to the irritation phenomena, as follows:

| Phenomenon | Abbreviation | Rating |
|---|---|---|
| Vesicles | V | 5 |
| Edema | E | 4 |
| Erythema | R | 3 |
| Flakiness | F | 2 |
| Dryness | D | 1 |
| Wrinkling | W | 1 |
| Translucency | T | 1 |
| Glassy | G | 1 |

A numerical value for each skin site was obtained by multiplying the score for the reaction with the rating for the phenomena occurred. The obtained values were summed and in this way a total number for the degree of irritation at that site was obtained.

As an example it can be mentioned that the total score for a site, for which R3, D2 and G1 are observed, would be as follows:

$$(3\times3)+(2\times1)+(1\times1)=12.$$

The tested substances were lauryl polyether sulphate as sodium salt, average number of 2 oxyethylene units indicated hereinafter as "A".

Lauryl-myristyl (70:30)—$(OC_2H_4)_xOCH_2COONa$, wherein x has an average value of 10, indicated hereinafter as "B". Five experiments were carried out with:

1. 100% A;
2. 95% A+5% B;
3. 88% A+12% B;
4. 80% A+20% B; and
5. 100% B, respectively.

All samples were tested in a concentration of 15% in water. The following table gives the mean results for the score of the skin irritation after 24 hours and after 48 hours for each of these experiments. In this respect it should still be remarked that a direct comparison of these results with those of the above discussed article from Seifen, Öle, Wachse is not possible. For it concerns here a different series of tests with different test persons. Therefore, the results are only comparable with each other within a series.

| | Mean score for skin irritation after | |
|---|---|---|
| Sample | 24 hours | 48 hours |
| 1. | 1.92 | 2.04 |
| 2. | 1.88 | 1.64 |
| 3. | 1.48 | 1.48 |
| 4. | 1.12 | 1.30 |
| 5. | 0.92 | 1.00 |

As mentioned already the present mixtures are particularly suitable as active detergents in compositions which are intended for prolonged or frequent contact with human hair or the human skin, such as shampoos, showering and bath compositions, liquid soap and agents for non-automatic dishwashing. Consequently, the invention also encompasses such compositions which for the rest can be composed in any usual way.

**Claims for the Contracting States: BE, CH, DE, FR, GB, IT, LI, LU, NL, SE**

1. A mixture of alkylether sulphate and alkylpolyglycol ether carboxylic acid or a salt thereof, particularly suitable for frequent or prolonged contact with human hair and skin, wherein the alkylpolyglycol ether carboxylic acid contains at least 7 oxyethylene units, characterized by the fact that the mixture contains 80—95% by weight of alkylether sulphate and 20—5% by weight of alkylpolyglycol ether carboxylic acid or a salt thereof of the formula

$$RO—(C_2H_4O)_x—CH_2COOM$$

wherein R is an alkyl residue having 10—20 carbon atoms, x is a number having an average value of 7—25 and M represents hydrogen, sodium, potassium, magnesium or an amine salt.

2. A mixture according to claim 1, characterized by the fact that the alkylpolyglycol ether carboxylate is a sodium salt which contains an alkyl residue having 12—18 carbon atoms and an average value of 9—18 oxyethylene units.

3. A mixture according to claim 1 or 2, characterized by the fact that it contains sodium lauryl ether sulphate having an average number of 2—3 oxyethylene units and the sodium salt of lauryl-myristyl

$$(70:30)—(OC_2H_4)_x—OCH_2COOH,$$

wherein x has an average value of 10.

4. Composition intended for frequent or prolonged contact with human hair or skin, characterized by the fact that it contains as detergent substance a mixture according to any of the claims 1—3.

5. Composition according to claim 4, characterized by the fact that it is a shampoo.

6. Composition according to claim 4, characterized by the fact that it is a showering or bath composition or a liquid soap.

7. Composition according to claim 4, characterized by the fact that it is an agent for non-automatic dishwashing.

**Claims for the Contracting State: AT**

1. A process for preparing a mixture of alkylether sulphate and alkylpolyglycol ether carboxylic acid or a salt thereof which is particularly suitable for frequent or prolonged contact with human hair and skin, wherein the alkylpolyglycol ether carboxylic acid contains at least 7 oxyethylene units, characterized by the fact that the mixture is prepared by mixing 80—95% by weight of alkylether sulphate with 20—5% by weight of alkylpolyglycol ether carboxylic acid or a salt thereof of the formula

$$RO—(C_2H_4O)_x—CH_2COOM$$

wherein R is an alkyl residue having 10—20 carbon atoms, x is a number having an average value of 7—25 and M represents hydrogen, sodium, potassium, magnesium or an amine salt.

2. A process according to claim 1, characterized by using as the alkylpolyglycol ether carboxylate a sodium salt which contains an alkyl residue of 12—18 carbon atoms and an average value of 9—18 oxyethylene units.

3. A process according to claim 1 or 2, characterized by preparing a mixture which contains sodium laurylether sulphate having an average of 2—3 oxyethylene units and the sodium salt of lauryl-myristyl

$$(70:30)—(OC_2H_4)_x—OCH_2COOH,$$

wherein x has an average value of 10.

4. A process for preparing a composition intended for frequent or prolonged contact with human hair and skin, characterized by incorporating therein as detergent substance a mixture prepared according to any of claims 1—3.

5. A process according to claim 4, characterized by the fact that the composition is prepared in the form of a shampoo.

6. A process according to claim 4, characterized by the fact that the composition is prepared in the form of a showering or bath composition or a liquid soap.

7. A process according to claim 4, characterized by the fact that the composition is prepared in the form of an agent for non-automatic dishwashing.

**Patentansprüche für die Vertragsstaaten: BE, CH, DE, FR, GB, IT, LI, LU, NL und SE**

1. Mischung von Alkyläthersulfat und Alkylpolyglykoläthercarbonsäure oder eines Salzes hievon, die zum häufigen oder längeren Kontakt mit menschlichem Haar und menschlicher Haut besonders geeignet ist, worin die Alkylpolyglykoläthercarbonsäure mindestens 7 Oxyäthyleneinheiten enthält, dadurch gekennzeichnet, daß die Mischung 80 bis 95 Gew.% Alkyläthersulfat und 20 bis 5 Gew.% Alkylpolyglykoläthercarbonsäure oder ein Salz hievon der Formel

$$RO—(C_2H_4O)_x—CH_2COOM,$$

worin R einen Alkylrest mit 10 bis 20 Kohlenstoffatomen bedeutet, x eine Zahl von durchschnittlich 7 bis 25 ist und M Wasserstoff, Natrium, Kalium, Magnesium oder ein Aminsalz darstellt, enthält.

2. Mischung nach Anspruch 1, dadurch gekennzeichnet, daß das Alkylpolyglykoläthercarboxylat ein Natriumsalz ist, das einen Alkylrest mit 12 bis 18 Kohlenstoffatomen und durchschnittlich 9 bis 18 Oxyäthyleneinheiten enthält.

3. Mischung nach Anspruch 1 oder 2, dadurch gekennzeichnet, daß sie Natriumlauryläthersulfat mit durchschnittlich 2 bis 3 Oxyäthyleneinheiten und das Natriumsalz von Lauryl-Myristyl

$$(70:30)—(OC_2H_4)_x—OCH_2COOH,$$

worin x einen durchschnittlichen Wert von 10 aufweist, enthält.

4. Zusammensetzung zum häufigen oder längeren Kontakt mit menschlichem Haar oder menschlicher Haut, dadurch gekennzeichnet, daß sie als Detergenssubstanz eine Mischung nach einem der Ansprüche 1 bis 3 enthält.

5. Zusammensetzung nach Anspruch 4, dadurch gekennzeichnet, daß sie ein Shampoo ist.

6. Zusammensetzung nach Anspruch 4, dadurch gekennzeichnet, daß sie ein Dusch- oder Bademittel oder eine Flüssigseife ist.

7. Zusammensetzung nach Anspruch 4, dadurch gekennzeichnet, daß sie ein Mittel zum nicht-automatischen Geschirrwaschen ist.

**Patentansprüche der Vertragsstaat: AT**

1. Verfahren zum Herstellen einer Mischung von Alkyläthersulfat und Alkylpolyglykoläthercarbonsäure oder eines Salzes hievon, die zum häufigen oder längeren Kontakt mit menschlichem Haar und

menschlicher Haut besonders geeignet ist, worin die Alkylpolyglykoläthercarbonsäure mindestens 7 Oxyäthyleneinheiten enthält, dadurch gekennzeichnet, daß die Mischung dadurch hergestellt wird, daß 80 bis 95 Gew.% Alkyläthersulfat und 20 bis 5 Gew.% Alkylpolyglykoläthercarbonsäure oder eines Salzes hievon der Formel

$$RO—(C_2H_4O)_x—CH_2COOM,$$

worin R einen Alkylrest mit 10 bis 20 Kohlenstoffatomen bedeutet, x eine Zahl von durchschnittlich 7 bis 25 ist und M Wasserstoff, Natrium, Kalium, Magnesium oder ein Aminsalz darstellt, gemischt werden.

2. Verfahren nach Anspruch 1, gekennzeichnet durch die Verwendung eines Natriumsalzes als Alkylpolyglykoläthercarboxylat, das einen Alkylrest mit 12 bis 18 Kohlenstoffatomen und durchschnittlich 9 bis 18 Oxyäthyleneinheiten enthält.

3. Verfahren nach Anspruch 1 oder 2, gekennzeichnet durch Herstellen einer Mischung, die Natriumlauryläthersulfat mit durchschnittlich 2 bis 3 Oxyäthyleneinheiten und das Natriumsalz von Lauryl-Myristyl

$$(70{:}30)—(OC_2H_4)_x—OCH_2COOH,$$

worin x einen durchschnittlichen Wert von 10 aufweist, enthält.

4. Verfahren zum Herstellen einer Zusammensetzung zum häufigen oder längeren Kontakt mit menschlichem Haar oder menschlicher Haut, dadurch gekennzeichnet, daß als Detergenssubstanz eine Mischung, hergestellt nach einem der Ansprüche 1 bis 3, eingearbeitet wird.

5. Verfahren nach Anspruch 4, dadurch gekennzeichnet, daß die Zusammensetzung in Form eines Shampoos hergestellt wird.

6. Verfahren nach Anspruch 4, dadurch gekennzeichnet, daß die Zusammensetzung in Form eines Dusch- oder Bademittels oder einer Flüssigseife hergestellt wird.

7. Verfahren nach Anspruch 4, dadurch gekennzeichnet, daß die Zusammensetzung in Form eines Mittels zum nicht-automatischen Geschirrwaschen hergestellt wird.

**Revendications pour les Etats Contractants: BE, CH, DE, FR, GB, IT, LI, LU, NL, SE**

1. Mélange d'alkyléthersulfate et d'acide alkylpolyglycoléther carboxylique ou un sel de celui-ci, convenant particulièrement pour des contacts fréquents ou prolongés avec les cheveux et la peau humaine, dans lequel l'acide alkylpolyglycoléther carboxylique contient au moins 7 motifs d'oxyéthylène, caractérisé en ce que le mélange contient de 80 à 95% en poids d'alkyléthersulfate et de 20 à 5% en poids d'acide alkylpolyglycoléthercarboxylique ou un sel de celui-ci, répondant à la formule:

$$RO—(C_2H_4O)_x—CH_2COOM$$

dans laquelle R est un radical alkyle de 10 à 20 atomes de carbone, *x* est un nombre d'une valeur moyenne de 7 à 25 et M représente un atome d'hydrogène, de sodium, de potassium ou de magnésium ou un sel d'amine.

2. Mélange selon la revendication 1, caractérisé en ce que l'alkylpolyglycoléther carboxylate est un sel de sodium contenant un radical alkyle de 12 à 18 atomes de carbone et ayant une valeur moyenne de 9 à 18 motifs d'oxyéthylène.

3. Mélange selon la revendication 1 ou 2, caractérisé en ce qu'il contient un lauryléthersulfate de sodium ayant un nombre moyen de 2 à 3 motifs d'oxyéthylène et le sel de sodium de lauryl-myristyle

$$(70{:}30)—(OC_2H_4)_x—OCH_2COOH$$

dans lequel *x* a une valeur moyenne de 10.

4. Composition destinée à des contacts fréquents ou prolongés avec les cheveux ou la peau humaine, caractérisée en ce qu'elle contient à titre de substance détergente, un mélange selon l'une quelconque des revendications 1 à 3.

5. Composition selon la revendication 4, caractérisée en ce qu'elle est un shampooing.

6. Composition selon la revendication 4, caractérisée en ce qu'elle est une composition pour la douche ou le bain ou un savon liquide.

7. Composition selon la revendication 4, caractérisée en ce qu'elle est un agent pour le lavage non automatique de la vaisselle.

**Revendications pour l'Etat Contractant: AT**

1. Procédé de préparation d'un mélange d'alkyléthersulfate et d'acide alkylpolyglycoléther carboxylique ou un sel de celui-ci, convenant particulièrement pour des contacts fréquents ou prolongés avec les cheveux et la peau humaine, dans lequel l'acide alkylpolyglycoléther carboxylique contient au

moins 7 motifs d'oxyéthylène, caractérisé en ce qu'on prépare le mélange en mélangeant 80 à 95% en poids d'alkyléthersulfate avec 20 à 5% en poids d'acide alkylpolyglycoléther carboxylique ou un sel de celui-ci, de formule

$$RO—(C_2H_4O)_x—CH_2COOM$$

dans laquelle R est un radical alkyle de 10 à 20 atomes de carbone, *x* est un nombre ayant une valeur moyenne de 7 à 25 et M représente un atome d'hydrogène, de sodium, de potassium ou de magnésium ou un sel d'amine.

2. Procédé selon la revendication 1, caractérisé en ce qu'on utilise, à titre d'alkylpolyglycoléther carboxylate, un sel de sodium qui contient un radical alkyle de 12 à 18 atomes de carbone et en moyenne de 9 à 18 motifs d'oxyéthylène.

3. Procédé selon la revendication 1 ou 2, caractérisé en ce qu'on prépare un mélange qui contient un lauryléthersulfate de sodium contenant en moyenne 2 à 3 motifs d'oxyéthylène et le sel de sodium de lauryl-myristyle

$$(70:30)—(OC_2H_4)_x—OCH_2COOH$$

dans lequel *x* a une valeur moyenne de 10.

4. Procédé de préparation d'une composition destinée à des contacts fréquents ou prolongés avec les cheveux et la peau humaine, caractérisé en ce qu'on incorpore à titre de substance détergente, un mélange préparé selon l'une quelconque des revendications 1 à 3.

5. Procédé selon la revendication 4, caractérisé en ce qu'on prépare la composition sous forme d'un shampooing.

6. Procédé selon la revendication 4, caractérisé en ce qu'on prépare la composition sous forme d'une composition de douche ou de bain ou d'un savon liquide.

7. Procédé selon la revendication 4, caractérisé en ce qu'on prépare la composition sous forme d'un agent de lavage de vaisselle non automatique.